# EUROPÄISCHE PATENTSCHRIFT

(11) **EP 2 535 040 B1**
(45) Veröffentlichungstag und Bekanntmachung des Hinweises auf die Patenterteilung: **19.04.2017**
(21) Anmeldenummer: 12168009.4
(22) Anmeldetag: 15.05.2012
(51) Int. Cl.: A61K 8/86, A61K 8/88, A61Q 11/00, A61Q 19/00, A61Q 15/00, A61Q 5/00, A61K 8/39, A61K 8/64, A61Q 17/00

(54) **Wirkstoffkombinationen aus (Epsilon-Polylysin und einem oder mehreren ethoxylierten und/oder propoxylierten Alkanolen**
Active agent combinations of (epsilon-polylysine and one or more ethoxylated and/or propoxylated alcohols
Combinaisons de substances actives à base d'epsilon-polylysine et d'un ou plusieurs alkanols éthoxylés et/ou propoxylés

(30) Priorität: 07.06.2011 DE 102011077040
(43) Veröffentlichungstag der Anmeldung: 19.12.2012
(73) Patentinhaber: Beiersdorf AG, 20253 Hamburg (DE)
(72) Erfinder: Klüter, Sabine, 58675 Hemer (DE); Traupe, Bernd, 24568 Kaltenkirchen (DE); Priebe, Laura, 22049 Hamburg (DE); Wöhrmann, Michael, 22851 Norderstedt (DE); Thiesen, Kathrin, 22301 Hamburg (DE)

(56) Entgegenhaltungen:
- WO-A1-2012/055585
- WO-A2-2009/150090
- FR-A1- 2 922 446
- JP-A- 62 221 616
- JP-A- 2004 026 743
- JP-A- 2004 035 461
- JP-A- 2004 317 769
- JP-A- 2007 153 791
- JP-A- 2009 108 065
- JP-A- 2011 079 874

## Beschreibung

Die vorliegende Erfindung betrifft Wirkstoffkombinationen aus ε-polylysin (Epsilon-Polylysin) und einem oder mehreren b) ein oder mehrere ethoxylierte oder propoxylierte Alkanolen, sowie kosmetische oder dermatologische Zubereitungen mit einem Gehalt an solchen Wirkstoffkombinationen sowie deren Verwendung als gegen Bakterien, Mycota und Viren wirksame Substanzen.

Der gesunde warmblütige Organismus, insbesondere die gesunde menschliche Haut, ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bakterien gehören zu den prokaryotischen Einzellern. Sie können grob nach ihrer Form (Kugel, Zylinder, gekrümmter Zylinder) sowie nach dem Aufbau ihrer Zellwand (grampositiv, gramnegativ) unterschieden werden. Feinere Unterteilungen tragen auch der Physiologie der Organismen Rechnung. So existieren aerobe, anaerobe sowie fakultativ anaerobe Bakterien. Manche Individuen sind in ihrer Eigenschaft als pathogene Keime von medizinischer Bedeutung, andere wiederum sind vollkommen harmlos.

Gegen Bakterien wirksame Substanzen sind seit geraumer Zeit bekannt. Der Begriff "Antibiotika" beispielsweise, der nicht auf alle antimikrobiell wirksamen Substanzen anwendbar ist, läßt sich auf das Jahr 1941 datieren, obwohl die ersten Erkenntnisse zum Penicillin bereits im Jahre 1929 gefunden wurden. Antibiotika im heutigen Sinne sind nicht für alle medizinischen, schon gar nicht kosmetische Anwendungen geeignet, da häufig auch der warmblütige Organismus, also etwa der erkrankte Patient, bei Anwendung auf irgendeine Weise in seinen Stoffwechselfunktionen beeinträchtigt wird.

Eine Aufgabe der vorliegenden Erfindung war also, den Stand der Technik in dieser Richtung zu bereichern, insbesondere also, Substanzen zur Verfügung zu stellen, welche gegen grampositive und/oder gramnegative Bakterien wirksam sind, ohne daß mit der Anwendung der Substanzen eine unvertretbare Beeinträchtigung der Gesundheit des Anwenders verbunden wäre.

Gramnegative Keime sind beispielsweise Escherichia coli, Pseudomonas-Arten sowie Enterobacteriaceen, wie etwa Citrobacter.

Auch grampositive Keime spielen in Kosmetik und Dermatologie eine Rolle. Bei der unreinen Haut beispielsweise sind neben anderen Einflüssen bakterielle Sekundärinfektionen von ätiologischer Bedeutung. Einer der wichtigsten Mikroorganismen, der in Zusammenhang mit unreiner Haut steht, ist Propionibacterium acnes.

Unreine Haut und/oder Komedonen beeinträchtigen das Wohlbefinden der Betroffenen aber selbst in leichten Fällen. Da praktisch jeder oder jede Jugendliche von unreiner Haut irgendeiner Ausprägung betroffen ist, besteht bei vielen Personen Bedarf, diesem Zustande abzuhelfen.

Eine besondere Aufgabe der vorliegenden Erfindung war es also, einen gegen unreine Haut bzw. Propionibacterium acnes wirksamen Stoff bzw. Stoffkombination zu finden.

Die vorliegende Erfindung betrifft in einer weiteren Ausführungsform kosmetische Desodorantien. Solche Formulierungen dienen dazu, Körpergeruch zu beseitigen, der entsteht, wenn der an sich geruchlose frische Schweiß durch insbesondere grampositive Mikroorganismen zersetzt wird. Den üblichen kosmetischen Desodorantien liegen unterschiedliche Wirkprinzipien zugrunde.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Entstehung des Schweißes unterbunden werden. Abgesehen von der Denaturierung der Hautproteine greifen die dafür verwendeten Stoffe aber, abhängig von ihrer Dosierung, drastisch in den Wärmehaushalt der Achselregion ein und sollten allenfalls in Ausnahmefällen angewandt werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. In der Praxis hat sich aber herausgestellt, daß die gesamte Mikroflora der Haut beeinträchtigt werden kann.

Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich. Die Nachteile beider Wirkstoffklassen lassen sich auf diesem Wege jedoch nicht vollständig beseitigen.

Schließlich kann Körpergeruch auch durch Duftstoffe überdeckt werden, eine Methode, die am wenigsten den ästhetischen Bedürfnissen des Verbrauchers gerecht wird, da die Mischung aus Körpergeruch und Parfümduft eher unangenehm riecht.

Allerdings werden die meisten kosmetischen Desodorantien, wie auch die meisten Kosmetika insgesamt, parfümiert, selbst wenn sie desodorierende Wirkstoffe beinhalten. Parfümierung kann auch dazu dienen, die Verbraucherakzeptanz eines kosmetischen Produktes zu erhöhen oder einem Produkt ein bestimmtes Flair zu geben.

Die Parfümierung wirkstoffhaltiger kosmetischer Mittel, insbesondere kosmetischer Desodorantien, ist allerdings nicht selten problematisch, weil Wirkstoffe und Parfümbestandteile gelegentlich miteinander reagieren und einander unwirksam machen können.

Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Eine weitere Aufgabe der vorliegenden Erfindung war es also, kosmetische Desodorantien zu entwickeln, die die Nachteile des Standes der Technik nicht aufweisen. Insbesondere sollten die Desodorantien die Mikroflora der Haut weitgehend schonen, die Zahl der Mikroorganismen aber, die für den Körpergeruch verantwortlich sind, selektiv reduzieren.

Weiterhin war es eine Aufgabe der Erfindung, kosmetische Desodorantien zu entwickeln, die sich durch gute Hautverträglichkeit auszeichnen. Auf keinen Fall sollten die desodorierenden Wirkprinzipien sich auf der Haut anreichern.

Eine weitere Aufgabe war, kosmetische Desodorantien zu entwickeln, welche mit einer möglichst großen Vielzahl an üblichen kosmetischen Hilfs- und Zusatzstoffen harmonieren, insbesondere mit den gerade in desodorierend oder antitranspirierend wirkenden Formulierungen bedeutenden Parfümbestandteilen.

Noch eine weitere Aufgabe der Erfindung war, kosmetische Desodorantien zur Verfügung zu stellen, welche über einen längeren Zeitraum, und zwar in der Größenordnung von mindestens einem halben Tag, wirksam sind, ohne daß ihre Wirkung spürbar nachläßt.

Schließlich war eine Aufgabe der vorliegenden Erfindung, desodorierende kosmetische Prinzipien zu entwickeln, die möglichst universell in die verschiedensten Darreichungsformen kosmetischer Desodorantien eingearbeitet werden können, ohne auf eine oder wenige spezielle Darreichungsformen festgelegt zu sein.

Pilze, auch Fungi, Mycota oder Mycobionten genannt, zählen im Gegensatze zu den Bakterien zu den Eucaryonten. Eucaryonten sind Lebewesen, deren Zellen (Eucyten) im Gegensatz zu denen der sogenannten Procaryonten (Procyten) über einen durch Kernhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis und andere Pityrosporum-bedingte Mycosen, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose und Skopulariopsidose), Systemmycosen (z.B. Chromomycose, Coccidiomycose, Histoplasmose).

Zu den pathogenen und fakultativ pathogenen Keimen gehören beispielsweise aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen. Eine Beteiligung von Pityrosporum ovale an der Entstehung von Psoriasis wird von der Fachwelt diskutiert.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche (Onychomykosen).

Ferner sind Superinfektionen der Haut durch Pilze und Bakterien nicht selten.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio-oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, die im weitesten Sinne mikrobiellen Befall verhindert, bekämpft oder abwehrt.

Es wurde überraschend gefunden, und darin liegt die Lösung dieser Aufgabe, daß Wirkstoffkombinationen umfassend:
a) ε-Polylysin (Epsilon-Polylysin), die einen Polymerisationsgrad von 2 bis 200 aufweisen, bevorzugt 10 bis 100, besonders bevorzugt von 23 bis 35
b) einen oder mehrere ethoxylierte oder propoxylierte Alkanole, welcher oder welche gewählt wird oder werden aus der Gruppe Polyethylenglykol-(20)-stearylether. (= Steareth-20), Polyethylenglykol-(100)-stearylether (= Steareth-100) sowie PPG-15 Stearyl Ether
synergistische antimikrobielle Wirkung aufweisen und den Nachteilen des Standes der Technik abhelfen.

L-Lysin ist durch folgende chemische Struktur gekennzeichnet: 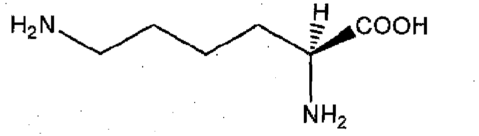 ε-Polylysin stellt ein Polymer dar, bei dem die ε-ständige Aminogruppe eines Lysinmoleküls mit der Säurefunktion eines weiteren Lysinmoleküls verknüpft ist

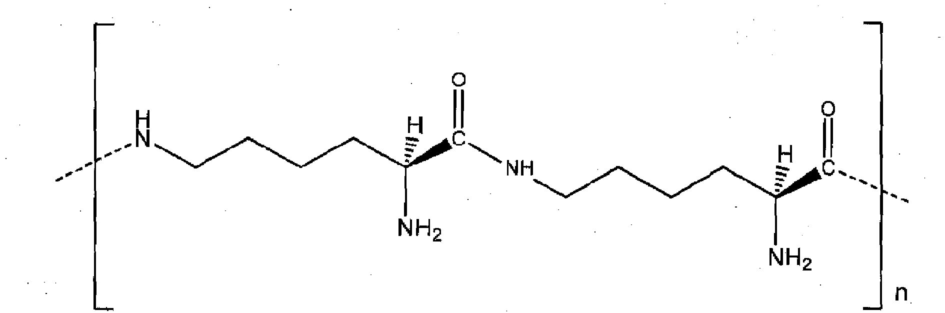

Durch den Index "n" (genauer gesagt: durch den Wert n/2) wird der Polymerisationsgrad des Polylysins definiert.

### Die INGI-Bezeichnung lautet Poly[imino[(2S)-2-amino-1-oxo-1,6-hexandiyl]], es hat die CAS-Nr. 28211-04-3

Das Homopolymer ε-Polylysin wird bekannterweise als antimikrobielles Agens gegen Hefen, Pilze und gram-positive und gram-negative Bakterien beschrieben und eingesetzt. Derzeit findet es als Konservierungsmittel, vor allem in Japan, Korea und den USA, Anwendung. Die Herstellung des ε-Polylysins erfolgt über einen natürlichen fermentativen Prozess,

### Referenzen:

1. Shima, S. and Sakai H. (1977). "Polylysine produced by Streptomyces". Agricultural and Biological Chemistry 41: 1807-1809.
2. Shima, S. et al. (1984). "Antimicrobial action of ε-poly-L-lysine". Journal of Antibiotics 37 (11): 1449-1455.
3. GRAS Notice No. GRN 000135
4. Hiraki, J. et al. (2003). "Use of ADME studies to confirm the safety of ε-polylysine as a preservative in food". Regulatory Toxicology and Pharmacology 37 (2): 328-340.
5. Hiraki, J. (1995). "Basic and applied studies on ε-polylysine". Journal of Antibacterial Antifungal Agents 23: 349-354

Erfindungsgemäß wird ε-Polylysin bevorzugt in kosmetischen oder dermatologischen Zusammensetzungen eingesetzt einem Gehalt von 0,0005- 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 -10,0 Gew.-%, besonders bevorzugt 0,02 - 5,0 Gew.-% an ε-Polylysin, ganz besonders vorteilhaft 0,05 - 0,5 Gew.-%, jeweils bezogen auf das Gesamtgewicht der Zusammensetzung.

ε-Polylysin läßt sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z.B. Nagellacke, Nagellackentferner, Nagelbalsame) und dergleichen.

m kann vorteilhaft aus dem Bereich von 1 bis 200, besonders vorteilhaft aus dem Bereich 10 bis 150 gewählt werden. Die Werte für m stellen allerdings gemittelte Werte dar, da sich herstellungsbedingt eine gewisse Verteilungsbreite des Ethoxylierungsgrades ergibt.

Wenn R¹ = H, wird m bevorzugt aus dem Zahlenbereich von 10 bis 150 gewählt.
Wenn R¹ = Methyl, wird m bevorzugt aus dem Zahlenbereich von 5 bis 50 gewählt.

Bevorzugte erfindungsgemäße ethoxylierte oder propoxylierte Alkanole stellen die Species Polyethylenglykol-(20)-stearylether (= Steareth-20), Polyethylenglykol-(100)-stearylether (= Steareth-100) sowie PPG-15 Stearyl Ether dar.

Erfindungsgemäß werden der oder die ethoxylierten oder propoxylierten Alkanole bevorzugt in kosmetischen oder dermatologischen Zusammensetzungen eingesetzt einem Gehalt von 0,0005 - 50,0 Gew.-%, insbesondere 0,01 - 20,0 Gew.-%, bezogen auf das Gesamtgewicht der Zusammensetzung bevorzugt sind. Vorteilhaft enthalten die Zusammensetzungen 0,02 - 10,0 Gew.-%, besonders bevorzugt 0,02 - 5,0 Gew.-% an den erfindungsgemäß verwendeten ethoxylierten oder propoxylierten Alkanole, ganz besonders vorteilhaft 0,1 - 5,0 Gew.-%, jeweils bezogen auf das Gesamtgewicht der Zusammensetzung.

Erfindungsgemäß besonders bevorzugt werden Gewichtsverhältnis aus
a) ε-Polylysin einerseits und
b) der Gesamtheit aus einem oder mehreren ethoxylierten oder propoxylierten Alkanole andererseits
von 10 : 1 bis 1 : 100, vorteilhaft von 5 : 1 bis 1 zu 20, insbesondere vorteilhaft von 1 : 1 bis 1 : 10 gewählt.

Die erfindungsgemäß verwendeten Wirkstoffkombinationen eignen sich besonders gut für die Verwendung als desodorierender Wirkstoff in kosmetischen Desodorantien sowie gegen unreine Haut und leichte Formen der Akne.

Ferner hat sich herausgestellt, daß die erfindungsgemäß verwendeten Wirkstoffkombinationen den Verderb organischer Substanz, insbesondere kosmetischer und dermatologischer Zubereitungen, durch den Befall mit grampositiven und gramnegativen Bakterien, Mycobionten und Viren verhindern können, wenn sie diesen Zubereitungen zugesetzt werden.

Die erfindungsgemäß verwendeten Einzelwirkstoffe lassen sich ohne Schwierigkeiten in gängige kosmetische oder dermatologische Formulierungen einarbeiten, vorteilhaft in Pumpsprays, Aerosolsprays, Cremes, Salben, Tinkturen, Lotionen, Nagelpflegeprodukte (z.B. Nagellacke, Nagellackentferner, Nagelbalsame) und dergleichen.

Vorteilhaft liegen die erfindungsgemäß verwendeten Einzelwirkstoffe in Form von Antischuppen-Shampoos vor.

Es ist auch möglich und gegebenenfalls vorteilhaft, die erfindungsgemäß verwendeten Einzelwirkstoffe mit anderen Wirkstoffen zu kombinieren, beispielsweise mit anderen antimikrobiell, antimycotisch, antiparasitär bzw. antiviral wirksamen Stoffen.

Es ist auch gegebenenfalls vorteilhaft, die Zusammensetzungen gemäß der Erfindung abzupuffern. Vorteilhaft ist ein pH-Bereich von 3,5 - 7,5. Besonders günstig ist es, den pH-Wert in einem Bereich von 4,0 - 6,5 zu wählen.

Die kosmetischen und/oder dermatologischen Formulierungen gemäß der Erfindung können wie üblich zusammengesetzt sein und zur Behandlung der Haut und/oder der Haare im Sinne einer dermatologischen Behandlung oder einer Behandlung im Sinne der pflegenden Kosmetik dienen. Sie können aber auch in Schminkprodukten in der dekorativen Kosmetik eingesetzt werden.

Zur Anwendung werden die kosmetischen und/oder dermatologischen Formulierungen gemäß der Erfindung in der für Kosmetika und Dermatika üblichen Weise auf die Haut und/oder die Haare in ausreichender Menge aufgebracht.

Vorteilhaft sind solche kosmetische und dermatologische Zubereitungen, die in der Form eines Sonnenschutzmittels vorliegen. Vorteilhaft enthalten diese zusätzlich mindestens einen UVA-Filter und/oder mindestens einen UVB-Filter und/oder mindestens ein anorganisches Pigment.

Kosmetische Zubereitungen gemäß der Erfindung zum Schutze der Haut vor UV-Strahlen können in verschiedenen Formen vorliegen, wie sie z.B. üblicherweise für diesen Typ von Zubereitungen eingesetzt werden. So können sie z.B. eine Lösung, eine Emulsion vom Typ Wasser-in-Öl (W/O) oder vom Typ Öl-in-Wasser (O/W), oder eine multiple Emulsionen, beispielsweise vom Typ Wasser-in-Öl-in-Wasser (W/O/W), ein Gel, eine Hydrodispersion, einen festen Stift oder auch ein Aerosol darstellen.

Die kosmetischen Zubereitungen gemäß der Erfindung können kosmetische Hilfsstoffe enthalten, wie sie üblicherweise in solchen Zubereitungen verwendet werden, z.B. Antioxidantien, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende und/oder feuchhaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Emulsionen sind gemäß der Erfindung vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte gemäß der Erfindung enthalten z.B. natürliche oder synthetische Wachse, Fettalkohole oder Fettsäureester. Bevorzugt werden Lippenpflegestifte sowie desodorierende Stifte ("Deo-Sticks").

Als Treibmittel für erfindungsgemäße, aus Aerosolbehältern versprühbare kosmetische oder dermatologische Zubereitungen sind die üblichen bekannten leichtflüchtigen, verflüssigten Treibmittel, z.B. Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können die erfindungsgemäßen Zubereitungen zudem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen.

Bei kosmetischen Zubereitungen zur Pflege der Haare handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die kosmetischen Zubereitungen enthalten Wirkstoffe und Hilfsstoffe, wie sie üblicherweise für diesen Typ von Zubereitungen zur Haarpflege und Haarbehandlung verwendet werden.

Als Hilfsstoffe dienen Konservierungsmittel, oberflächenaktive Substanzen, Substanzen zum Verhindern des Schäumens, Emulgatoren, Verdickungsmittel, Fette, Öle, Wachse, organische Lösungsmittel, Bakterizide, Parfüme, Farbstoffe oder Pigmente, deren Aufgabe es ist, die Haare oder die Zubereitung selbst zu färben, Elektrolyte, Zubereitungen gegen das Fetten der Haare.

Kosmetische Zubereitungen, die ein Shampoonierungsmittel oder eine Wasch-, Dusch- oder Badezubereitung darstellen, enthalten vorzugsweise mindestens eine anionische, nichtionische oder amphotere oberflächenaktive Substanz oder Gemische daraus, mindestens eine dialkylsubstituierte Carbonsäure im wäßrigen Medium und Hilfsmittel, wie sie üblicherweise dafür verwendet werden.

Die folgenden Beispiele sollen die Verkörperungen der vorliegenden Erfindungen verdeutlichen. Die Angaben beziehen sich stets auf Gewichts-%, sofern nicht andere Angaben gemacht werden.

**Beispielrezepturen : Ceteareth-20**

| | **Nr. 1** | **Nr. 2** |
|---|---|---|
| **Chemische Bezeichnung** | **Gew.-%** | **Gew.-%** |
| Polyoxyethylen(20)cetylstearylether | 3,0 | 4,0 |
| Polyoxyethylen(12)cetylstearylether | 0,5 | - |
| Glycerinstearat | 3,0 | 3,0 |
| Cetylstearylalkohol | 0,5 | - |
| Cetylpalmitat | 0,5 | - |
| Capryl-Caprinsäureester | 4,0 | 3,5 |
| Di-n-Octylether | 5,0 | 5,0 |
| Polyethylenglycol(150)distearat | - | 1,0 |
| Glycerin | 4,0 | 2,0 |
| e-polylysin | 0,1 | 0,25 |
| Parfüm | q.s. | q.s. |
| Wasser | ad 100,00 | ad 100,00 |

| | **Nr. 3** | **Nr. 4** |
|---|---|---|
| **Chemische Bezeichnung** | **Gew.-%** | **Gew.-%** |
| Polyoxyethylen(20)stearylether | 1,0 | 0,50 |
| Polyoxyethylen(20)cetylstearylether | 1,5 | 2,5 |
| Polyoxyethylen(12)cetylstearylether | 1,0 | 0,5 |
| Glycerinstearat | 2,0 | 3,0 |
| Cetylstearylalkohol | 0,5 | - |
| Cetylpalmitat | 0,5 | - |
| Capryl-Caprinsäureester | 3,0 | 3,5 |
| Di-n-Octylether | 2,0 | 2,0 |
| C12-15 Alkylbenzoat | 2,0 | 2,0 |
| Glycerin | 4,0 | 2,0 |
| ε-Polylysin | 0,1 | 0,25 |
| Parfüm | q.s. | q.s. |
| Wasser | ad 100,00 | ad 100,00 |

**Roll-on Deodorant**

| | **Nr. 1** | **Nr. 2** |
|---|---|---|
| **Chemische Bezeichnung** | **Gew.- %** | **Gew.- %** |
| Polyethylenglykol(21)stearylether | 2,5 | 1,5 |
| Polyethylenglykol(2)stearylether | 1,5 | 2,5 |
| Polypropylenglykol(15)stearylether | 3 | 4 |
| Trinatriumsalz der Ethylendiamintetraessigsäure (20% wäßr. Lösung) | 1,5 | 1,5 |
| Parfum, Antioxidantien | q.s. | q.s. |
| ε-polylysin | 0,1 | 0,5 |
| Wasser | ad 100 | ad 100 |

**Roll-on Deodorant**

| | **Nr. 3** | **Nr. 4** |
|---|---|---|
| **Chemische Bezeichnung** | **Gew.- %** | **Gew.- %** |
| Polyethylenglykol(21)stearylether | 2,5 | 1,5 |
| Polyethylenglykol(2)stearylether | 1,5 | 2,5 |
| Polyoxyethylen(20)stearylether | 0,5 | 0,5 |
| Trinatriumsalz der Ethylendiamintetraessigsäure (20% wäßr. Lösung) | 1,5 | 1,5 |
| Parfum, Antioxidantien | q.s. | q.s. |
| ε-Polylysin | 0,1 | 0,5 |
| Wasser | ad 100 | ad 100 |

| | **Nr. 5** | **Nr. 6** |
|---|---|---|
| **Chemische Bezeichnung** | **Gew.-%** | **Gew.-%** |
| Polyoxyethylen(20)stearylether | 1,0 | 0,50 |
| Polyoxyethylen(20)cetylstearylether | 1,5 | 2,5 |
| Polyoxyethylen(12)cetylstearylether | 1,0 | 0,5 |
| Glycerinstearat | 2,0 | 3,0 |
| Cetylstearylalkohol | 0,5 | - |
| Cetylpalmitat | 0,5 | - |
| Capryl-Caprinsäureester | 3,0 | 3,5 |
| Di-n-Octylether | 2,0 | 2,0 |
| C12-15 Alkylbenzoat | 2,0 | 2,0 |
| Glycerin | 4,0 | 2,0 |
| ε-Polylysin | 0,1 | 0,25 |
| Parfüm | q.s. | q.s. |
| Wasser | ad 100,00 | ad 100,00 |

**Anti Acne Face Cream**

| | **Nr. 7** | **Nr. 8** |
|---|---|---|
| **Chemische Bezeichnung** | **Gew.-%** | **Gew.-%** |
| Polyoxyethylen(20)stearylether | 1,0 | 0,50 |
| Glyceryl Caprylat | 0,5 | - |
| Polyglyceryl 2-Caprat | 0,5 | 0,5 |
| Behenylalkohol | 5,0 | 4,0 |
| Cetylstearylalkohol | 1,5 | 3,0 |
| Butylenglykol | 4,0 | 5,0 |
| Zinkoxid | 10,0 | 5,0 |
| Panthenol | - | 0,2 |
| Bisabolol | 0,1 | - |
| Glycerin | - | 1,0 |
| Acrylates/C10-30 Alkyl Acrylat | 0,4 | 0,5 |
| Xanthan Gummi | 0,25 | 0,15 |
| ε-Polylysin | 0,1 | 0,25 |
| Phenoxyethanol | 0,5 | 0,4 |
| Methylisothiazolin | 0,05 | - |
| Methylparaben | - | 0,2 |
| Parfüm | q.s. | q.s. |
| Wasser | ad 100,00 | ad 100,00 |

| | **Nr. 9** | **Nr. 10** |
|---|---|---|
| **Chemische Bezeichnung** | **Gew.-%** | **Gew.-%** |
| Polyoxyethylen(20)stearylether | 1,0 | 1,0 |
| Glyceryl Caprylat | 0,5 | - |
| Polyglyceryl 2-Caprat | 0,5 | 0,5 |
| Myristylalkol | 1,0 | 1,0 |
| Cetylstearylalkohol | 1,5 | 2,0 |
| Butylenglykol | 2,0 | 3,0 |
| Cyclomethicon | 4,0 | 3,0 |
| Dimethicon | 1,0 | 1,5 |
| Phenoxyethanol | 0,3 | 0,5 |
| Methylisothiazolin | 0,05 | 0,05 |
| Glycerin | 5,0 | 3,0 |
| Arcrylates/C10-30 Alkyl Acrylat | 0,4 | 0,5 |
| Carbomer | 0,25 | 0,2 |
| ε-Polylysin | 0,1 | 0,25 |
| Parfüm | q.s. | q.s. |
| Wasser | ad 100,00 | ad 100,00 |

### Wirksamkeitsnachweis

### Bakteriensuspensionstest

### Vorbereitung der Bakteriensuspension:

Corynebacterium. jeikeium (DSM 7171) wurde aus einem Kryoröhrchen (EN 12353) auf einer Agarplatte (C+T-Agar) ausgestrichen und bei 30 °C 48 h inkubiert. Anschließend wurde eine zweite Passage angelegt.

Mit der zweiten Passage wurden 10 mL AC-Medium und 5 g Glasperlen (4 mm Durchmesser) angeimpft und 3 min geschüttelt. Danach wurde dieser Ansatz auf eine OD₆₀₀ von 0,08 eingestellt und anschließend 1:50 mit AC-Medium verdünnt (Keimzahl 2*10⁵). Diese Bakteriensuspension wurde anschließend für den Suspensionsest eingesetzt.

### Vorbereitung der Wirkstofflösung:

Die Rohstoffe wurden bei 80 °C im Wasserbad erwärmt und anschließend anteilig mit PE-Wasser zur Herstellung der Emulgator/Wasser-Lösungen versetzt. Für die Kombinationen mit ε-polylysin wurde festes ε-polylysin in der Rohstoff/Wasser-Mischung gelöst. Zur Kontrolle wurde eine Probe alleinig mit ε-polylysin in Wasser hergestellt.

### Test:

Zu 500 µL der vorgelegten Wirkstofflösung wurden 500 µl Bakteriensuspension hinzugegeben und auf einem Thermoschüttler (30°C, 1200 rpm) inkubiert. Nach 20, 60 und 180 min wurden aus dem Ansatz jeweils 100 µL entnommen und mit 900 µL AC-Medium verdünnt. Diese Suspension diente zur Ausplattierung auf Agar-Platten. Bei der Kontrolle wurde auch zum Zeitpunkt T = 0 min eine Probe genommen. Zum Schluss wurden die Platten für 48 h bei 30°C inkubiert und anschließend mit Hilfe des *CounterMat* ausgewertet.

### 1. Ergebnis

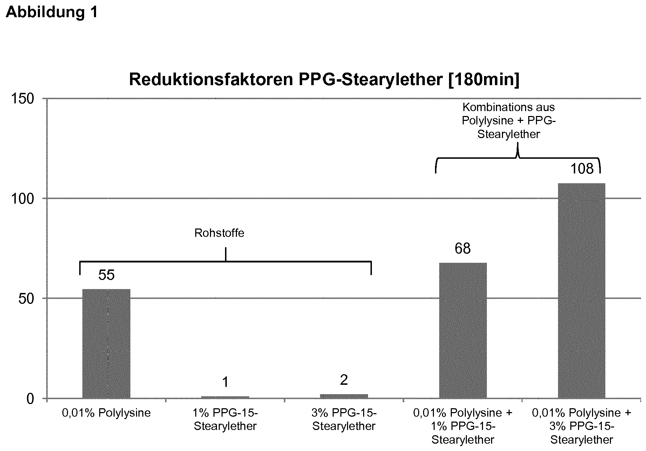

### Siehe Abbildung 1

Durch die Kombination von PPG-15 Stearyl Ether mit ε-polylysin kam es zu einer höheren Bakterien-Absterberate als mit dem alleinigen antimikrobiellen Wirkstoff ε-Polylysine. Die Reduktionsfaktoren sind in der Abbildung dargestellt.

### 2. Ergebnis

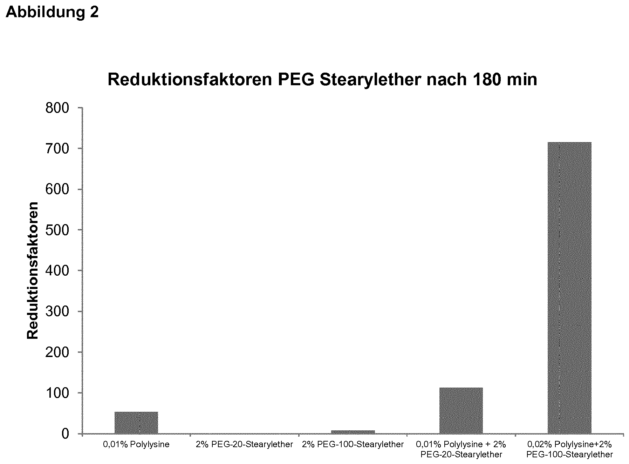

### Siehe Abbildung 2

Durch die Kombination von PEG-20-Stearylether bzw. PEG-100-Stearylether mit ε-Polylysine kam es zu einer stärkeren Bakterien-Absterberate als mit dem alleinigen antimikrobiellen Wirkstoff ε-polylysin. Die Reduktionsfaktoren sind in der Abbildung dargestellt.

## Patentansprüche

1. Wirkstoffkombinationen umfassend:
a) ε-Polylysin (Epsilon-Polylysin), die einen Polymerisationsgrad von 2 bis 200 aufweisen, bevorzugt 10 bis 100, besonders bevorzugt von 23 bis 35
b) einen oder mehrere ethoxylierte oder propoxylierte Alkanole, welcher oder welche gewählt wird oder werden aus der Gruppe Polyethylenglykol-(20)-stearylether (= Steareth-20), Polyethylenglykol-(100)-stearylether (= Steareth-100) sowie PPG-15 Stearyl Ether.

2. Wirkstoffkombinationen nach Anspruch 1,
bei denen Gewichtsverhältnisse aus
a) ε-Polylysin einerseits und
b) der Gesamtheit aus einem oder mehreren ethoxylierten oder propoxylierten Alkanolen andererseits von 10 : 1 bis 1 : 100, vorteilhaft von 5 : 1 bis 1 zu 20, insbesondere vorteilhaft von 1 : 1 bis 1 : 10 gewählt werden.

3. Kosmetische Zubereitungen, enthaltend Wirkstoffkombinationen nach einem der vorstehenden Ansprüche.

4. Zubereitungen nach Anspruch 3, in denen ε-Polylysin in Konzentrationen von 0,0005 - 50,0 Gew.-%, insbesondere 0,01 - 20,0 Gew.-%, bezogen auf das Gesamtgewicht der Zusammensetzung eingesetzt werden.

5. Zubereitungen nach Anspruch 3 oder 4, in denen der oder die ethoxylierten oder propoxylierten Alkanole in Konzentrationen von 0,0005 - 50,0 Gew.-%, insbesondere 0,01 - 20,0 Gew.-%, bezogen auf das Gesamtgewicht der Zusammensetzung eingesetzt werden.

## Claims

1. Active ingredient combinations comprising:
a) ε-polylysine (epsilon-polylysine) having a degree of polymerization of 2 to 200, preferably 10 to 100, particularly preferably 23 to 35
b) one or more ethoxylated or propoxylated alkanols, which is/are selected from the group of polyethylene glycol-20 stearyl ether (= Steareth-20), polyethylene glycol-100 stearyl ether (= Steareth-100) and PPG-15 stearyl ether.

2. Active ingredient combinations according to Claim 1, in which ratios by weight of
a) ε-polylysine on the one hand and
b) the totality of one or more ethoxylated or propoxylated alkanols on the other hand are selected from 10:1 to 1:100, advantageously from 5:1 to 1:20, particularly advantageously from 1:1 to 1:10.

3. Cosmetic preparations comprising active ingredient preparations according to either of the preceding claims.

4. Preparations according to Claim 3, in which ε-polylysine are used at concentrations of 0.0005 - 50.0% by weight, particularly 0.01 - 20.0% by weight, based on the total weight of the composition.

5. Preparations according to Claim 3 or 4, in which the ethoxylated or propoxylated alkanol(s) are used at concentrations of 0.0005 - 50.0% by weight, particularly 0.01 - 20.0% by weight, based on the total weight of the composition.

## Revendications

1. Combinaisons d'agents actifs, comprenant :
a) de l'ε-polylysine (epsilon-polylysine), qui présente un degré de polymérisation de 2 à 200, de préférence de 10 à 100, de manière particulièrement préférée de 23 à 35,
b) un ou plusieurs alcanols éthoxylés ou propoxylés, qui sont choisis dans le groupe constitué par l'éther stéarylique de polyéthylène glycol-(20) (= stéareth-20), l'éther stéarylique de polyéthylène glycol-(100) (= stéareth-100), ainsi que l'éther stéarylique de PPG-15.

2. Combinaisons d'agents actifs selon la revendication 1, dans lesquelles les rapports en poids entre
a) l'ε-polylysine d'un côté et
b) la totalité du ou des alcanols éthoxylés ou propoxylés d'un autre côté, sont choisis de 10:1 à 1:100, avantageusement de 5:1 à 1 sur 20, de manière particulièrement avantageuse de 1:1 à 1:10.

3. Préparations cosmétiques, contenant des combinaisons d'agents actifs selon l'une quelconque des revendications précédentes.

4. Préparations selon la revendication 3, dans lesquelles l'ε-polylysine est utilisée en concentrations de 0,0005 à 50,0 % en poids, notamment de 0,01 à 20,0 % en poids, par rapport au poids total de la composition.

5. Préparations selon la revendication 3 ou 4, dans lesquelles le ou les alcanols éthoxylés ou propoxylés sont utilisés en concentrations de 0,0005 à 50,0 % en poids, notamment de 0,01 à 20,0 % en poids, par rapport au poids total de la composition.
